# EUROPEAN PATENT SPECIFICATION

(11) **EP 1 534 137 B1**
(45) Date of publication and mention of the grant of the patent: **11.10.2017**
(21) Application number: 03749207.1
(22) Date of filing: 26.08.2003
(51) Int. Cl.: A61B 8/12

(54) **SYSTEM AND METHOD FOR IDENTIFYING A VASCULAR BORDER**
SYSTEM UND VERFAHREN ZUR IDENTIFIZIERUNG EINER GEFÄSSGRENZE
SYSTEME ET PROCEDE D'IDENTIFICATION D'UN CONTOUR VASCULAIRE

(30) Priority: 26.08.2002 US 406183 P; 26.08.2002 US 406254 P; 26.08.2002 US 406184 P; 26.08.2002 US 406185 P; 26.08.2002 US 406234 P
(43) Date of publication of application: 01.06.2005
(73) Proprietor: THE CLEVELAND CLINIC FOUNDATION, Cleveland, OH 44195 (US)
(72) Inventor: KLINGENSMITH, Jon, D., El Dorado Hills, California 95762 (US); VINCE, Geoffrey, D., Avon Lake, OH 44012 (US); NAIR, Anuja, Cleveland Heights, OH 44106 (US); KUBAN, Barry, D., Avon Lake, OH 44012 (US)
(74) Representative: de Haan, Poul Erik
(86) International application number: PCT/US2003/027075
(87) International publication number: WO 2004/017823

(56) References cited:
- US-A- 6 148 095
- US-B1- 6 381 350
- US-B1- 6 381 350
- TAINE M-C ET AL: "Segmentation of cardiac and vascular ultrasound images with extension to border kinetics" ULTRASONICS SYMPOSIUM, 1994. PROCEEDINGS., 1994 IEEE CANNES, FRANCE 1-4 NOV. 1994, NEW YORK, NY, USA,IEEE, US, vol. 3, 1 November 1994 (1994-11-01), pages 1773-1776, XP010139892 ISBN: 978-0-7803-2012-3
- PATRICK BRIGGER ET AL: "B-Spline Snakes: A Flexible Tool for Parametric Contour Detection" IEEE TRANSACTIONS ON IMAGE PROCESSING, IEEE SERVICE CENTER, PISCATAWAY, NJ, US, vol. 9, no. 9, 1 September 2000 (2000-09-01), XP011025647 ISSN: 1057-7149
- BRIGGER P ET AL: "B-spline snakes and a JAVA interface: an intuitive tool for general contour outlining" IMAGE PROCESSING, 1998. ICIP 98. PROCEEDINGS. 1998 INTERNATIONAL CONFERENCE ON CHICAGO, IL, USA 4-7 OCT. 1998, LOS ALAMITOS, CA, USA,IEEE COMPUT. SOC, US, vol. 2, 4 October 1998 (1998-10-04), pages 277-281, XP010308592 ISBN: 978-0-8186-8821-8

## Description

### BACKGROUND OF THE INVENTION

### 1. Field of the Invention

The present invention relates to vascular borders, or more particularly, to a system and method of using a first vascular image (or control points located therein) to identify a border on a second vascular image.

### 2. Description of Related Art

The present invention relates to medical imaging arts. It finds particular application to a system and method of identifying a border in an intra-vascular ultrasound (IVUS) image. It should be appreciated that while the present invention is described in terms of identifying a luminal and medial-adventitial border on an IVUS image, the present invention is not so limited. Thus, for example, identifying any border (or boundary) in any vascular image is within the scope of the present invention.

Ultrasonic imaging of portions of a patient's body provides a useful tool in various areas of medical practice for determining the best type and course of treatment. Imaging of the coronary vessels of a patient by ultrasonic techniques can provide physicians with valuable information. For example, the image data may show the extent of a stenosis in a patient, reveal progression of disease, help determine whether procedures such as angioplasty or atherectomy are indicated or whether more invasive procedures may be warranted.

In a typical ultrasound imaging system, an ultrasonic transducer is attached to the end of a catheter that is carefully maneuvered through a patient's body to a point of interest such as within a blood vessel. The transducer may be a single-element crystal or probe that is mechanically scanned or rotated back and forth to cover a sector over a selected angular range. Acoustic signals are then transmitted and echoes (or backscatter) from these acoustic signals are received. The backscatter data can be used to identify the type or density of a scanned tissue. As the probe is swept through the sector, many acoustic lines are processed building up a sector-shaped image of the patient. After the data is collected, an image of the blood vessel (i.e., an IVUS image) is reconstructed using well-known techniques. This image is then visually analyzed by a cardiologist to assess the vessel components and plaque content.

A typical analysis includes determining the size of the lumen and amount of plaque in the vessel. This is performed by generating an image of the vessel (e.g., an IVUS image) and manually drawing contoured boundaries on the image where the clinician believes the luminal and the medial-adventitial borders are located. This is a very time consuming process. Furthermore, this process is made more difficult when multiple images are being analyzed (e.g., to recreate a 3D vascular image, etc.) or the images are of poor quality (e.g., making the boundaries more difficult to see).

US patent 6,381,350 B1 discloses an intravascular ultrasound (IVUS) analysis system and method which determines luminal and medial-adventitial boundaries of a blood vessel. To determine the luminal boundary of a vessel, a user selects boundary points on the image believed to be locations of the luminal boundary. A boundary contour is generated based on the boundary points. The boundary contour is then optimized by adjusting the boundary points based on a radially determined edge of the luminal boundary performed on the image in polar format. Using interpolation a contour is automatically generated on other images and the interpolated contour is optimized.

Thus, it would be advantageous to have a system and method of identifying a border on a vascular image that overcomes at least one of these drawbacks.

### SUMMARY OF THE INVENTION

The present invention provides a system and method of using a first vascular image, or more particularly a plurality of control points located thereon, to identify a border on a second vascular image. Embodiments of the present invention operate in accordance with an intra-vascular ultrasound (IVUS) device and a computing device electrically connected thereto. Specifically, to produce data for use in one embodiment of the present invention, an IVUS console is electrically connected to a computing device and a transducer via a catheter. The transducer is inserted into a blood vessel of a patient and used to gather IVUS data (i.e., blood-vessel data, or data that can be used to identify the shape of a blood vessel, its density, its composition, etc.). The IVUS data is then provided to (or acquired by) the IVUS console, where it is used to produce an IVUS image of the vessel.

The IVUS data (or multiple sets thereof) is then provided to (or acquired by) the computing device. In one embodiment of the present invention, the computing device includes a plurality of applications operating thereon -- i.e., a border-detection application, an extrapolation application, and an active-contour application. These applications are used to (i) identify a border and control points on a first IVUS image (i.e., any IVUS image), (ii) extrapolate the control points to a second IVUS image (i.e., another IVUS image), (iii) identify a border on the second IVUS image, and (iv) adjust the border on the second IVUS image in accordance with at least one factor.

Specifically, the border-detection application is adapted to identify a border on a vascular image (e.g., an IVUS image). In one embodiment of the present invention, this is accomplished by analyzing the IVUS image, or IVUS data that corresponds to the IVUS image, to determine certain gradients located therein. This is because borders of vascular objects can be identified by a change in pixel color (e.g., light-to-dark, dark-to-light, shade1-to-shade2, etc). Once the border is identified, the border-detection application is used to identify at least one control point (i.e., a starting-control point) on the identified border. The extrapolation application is then used to identify at least one control point (i.e., an additional control point) on at least one other IVUS image. In a preferred embodiment of the present invention, this is done by extrapolating the previously identified control point (i.e., the starting-control point) to at least one other IVUS image. Once the control point(s) is extrapolated, the extrapolating application is adapted to identify (or approximate) a border that passes through the extrapolated point(s).

The active-contour application is then used to adjust the approximated border (i.e., the border passing through the extrapolated point(s)) to more closely match the actual border of the vascular object. In doing so, the active-contour application may consider, or take into account at least (i) image gradients (i.e., gradient factor), (ii) the proximity of the border to each extrapolated point (i.e., continuity or control-point factor), and/or (iii) border curvature or smoothness (i.e., curvature or boundary factor). Specifically, the gradient factor can be used to adjust the border if the neighboring pixels (as opposed to the pixels of the border) include border characteristics (e.g., a dark-to-light transition, etc.). In other words, if the neighboring pixels include border-like characteristics (or at least more so than the pixels forming the border), then the border is adjusted. The continuity factor and the curvature factor can be used to ensure that the border passes through each extrapolated point and does not include any sharp transitions (e.g., corners, etc.), respectively. In one embodiment of the present invention, the active-contour application is further adapted to adjust related borders on adjacent images if the boarder is manually adjusted.

A more complete understanding of the system and method of identifying a border on an IVUS image will be afforded to those skilled in the art, as well as a realization of additional advantages and objects thereof, by a consideration of the following detailed description of the preferred embodiment. Reference will be made to the appended sheets of drawings which will first be described briefly.

### BRIEF DESCRIPTION OF THE DRAWINGS

Figure 1 illustrates a vascular-border-identification system in accordance with one embodiment of the present invention.
Figure 2 illustrates and exemplary intra-vascular ultrasound (IVUS) image.
Figure 3 illustrates a plurality of borders that can be identified in an IVUS image.
Figure 4 illustrates a plurality of control points on one of the borders depicted in Figure 3.
Figure 5 illustrates how a plurality of 2D vascular images can be used to generate a 3D vascular image.
Figure 6 illustrates how the control points from a first image (e.g., the image depicted in Figure 4) can be extrapolated onto a second image.
Figure 7 illustrates a vascular image including a luminal boundary, a medial-adventitial boundary, and a plaque component located therebetween.
Figure 8 illustrates a method of identifying a border of a vascular object in accordance with one embodiment of the present invention.

### DETAILED DESCRIPTION OF THE PREFERRED EMBODIMENT

The present invention provides a system and method of using a first vascular image, or more particularly a plurality of control points located thereon, to identify a border on a second vascular image. In the detailed description that follows, like element numerals are used to describe like elements illustrated in one or more figures.

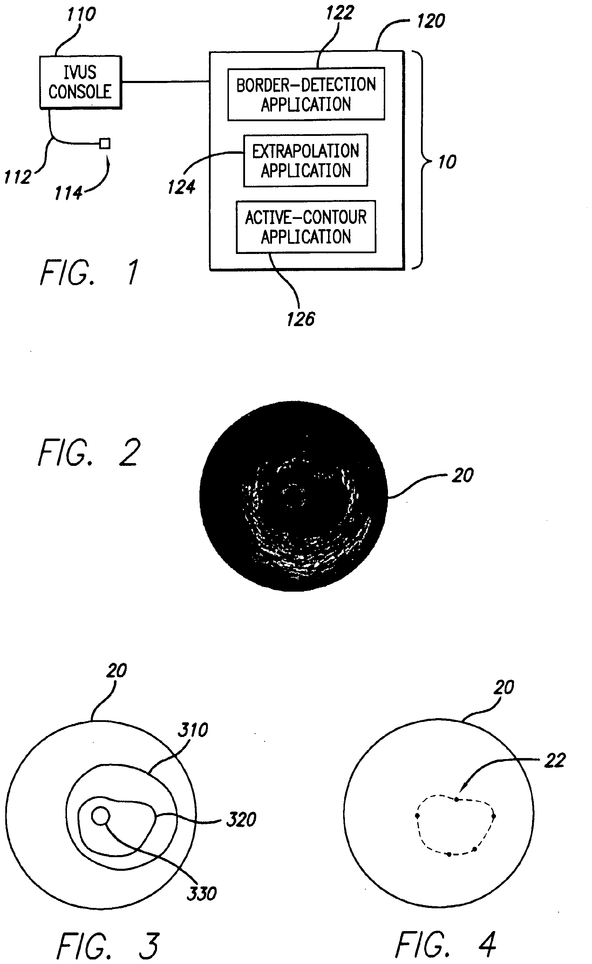

Embodiments of the present invention operate in accordance with an intra-vascular ultrasound (IVUS) device and a computing device electrically connected thereto. Figure 1 illustrates a vascular-border-identification system 10 in accordance with one embodiment of the present invention. Specifically, an IVUS console 110 is electrically connected to a computing device 120 and a transducer 114 via a catheter 112. The transducer 114 is inserted into a blood vessel of a patient (not shown) and used to gather IVUS data (i.e., blood-vessel data, or data that can be used to identify the shape of a blood vessel, its density, its composition, etc.). The IVUS data is then provided to (or acquired by) the IVUS console 110, where it is used to produce an IVUS image of the vessel.

More particularly, IVUS data is typically gathered in segments, either through a rotating transducer or an array of circumferentially positioned transducers, where each segment represents an angular portion of an IVUS image. Thus, it takes a plurality of segments (or a set of IVUS data) to image an entire cross-section of a vascular object. Furthermore, multiple sets of IVUS data are typically gathered from multiple locations within a vascular object (e.g., by moving the transducer linearly through the vessel). These multiple sets of data can then be used to create a plurality of two-dimensional (2D) images or one three-dimensional (3D) image. It should be appreciated that the present invention is not limited to the use of an IVUS device (or the acquisition of IVUS data), and may further include using thermographic devices, optical devices (e.g., an optical coherence tomography (OCT) console), MRI devices, or any vascular imaging devices generally known to those skilled in the art. It should further be appreciated that the computing device depicted in Figure 1 includes, but its not limited to, personal computers or any other data-processing devices (general purpose or application specific) that are generally known to those skilled in the art.

The IVUS data (or multiple sets thereof) is then provided to (or acquired by) the computing device 120. In one embodiment of the present invention, the computing device 120 includes a plurality of applications operating thereon -- i.e., a border-detection application 122, an extrapolation application 124, and an active-contour application 126. These applications are used to (i) identify a border and control points on a first IVUS image (i.e., any IVUS image), (ii) extrapolate the control points to a second IVUS image (i.e., another IVUS image), (iii) identify a border on the second IVUS image, and (iv) adjust the border on the second IVUS image. It should be appreciated that the number and/or location of the applications depicted in Figure 1 are not intended to limit the present invention, but are merely provided to illustrate the environment in which the present invention operates. Thus, for example, using a single application to perform the application functions, as discussed herein, or remotely locating at least one of the applications (in whole or in part) is within the scope of the present invention. It should further be appreciated that, while the present invention is discussed in terms of singularities (e.g., identifying a border on one IVUS image, extrapolating control points to another IVUS image, etc.), the present invention is not so limited. In fact, the present invention is particularly useful if it is used on a plurality of IVUS images (e.g., identifying borders on every fifth IVUS image, extrapolating control points from the fifth IVUS image to the next four IVUS images, etc.). It should also be appreciated that the terms "first" and "second," as those terms are used herein, are used broadly to identify any two IVUS images. Thus, the phrase "second IVUS image" may be used to identify an IVUS image distinct from a first IVUS image (as opposed to the second IVUS image in a series of IVUS images).

Vascular objects include several identifiable borders. For example, the luminal border demarcates the blood-intima interface and the medial-adventitial border demarcates the external elastic membrane (the boundary between the media and adventitia). By identifying these borders, the plaque-media complex, which is located there between, can be analyzed and/or calculated. It should be appreciated that the present invention is not limited to the identification of any particular border, and includes all vascular boundaries generally known to those skilled in the art.

Referring back to Figure 1, the border-detection application 122 is adapted to identify a border on a vascular image (e.g., an IVUS image). In one embodiment of the present invention, this is performed by analyzing the IVUS image, or IVUS data that corresponds the IVUS image, to determine certain gradients located therein. This is because borders of vascular objects can be identified by a change in pixel color (e.g., light-to-dark, dark-to-light, shade1-to-shade2, etc).

For example, Figure 2 illustrates an exemplary IVUS image 20 of a vascular object. Starting from the center and working outward, the catheter can be identified by the first light-to-dark transition (or gradient). The catheter border is further identified in Figure 3 (i.e., 330). Referring back to Figure 2, and continuing outward, the next dark-to-light transition (or gradient) identifies the luminal border (i.e., see Figure 3, 320). The medial-adventitial border can then be identified by going outward from the luminal border until the next dark-to-light transition (or gradient) is found (see Figure 3, 310). It should be appreciated that because the IVUS image is constructed using gray-scales, it may be necessary to utilize an algorithm and/or at least one threshold value to identify precisely where the image changes from light to dark (or vice versa). However, it should further be appreciated that the present invention is not limited to any particular algorithm for identifying the aforementioned transitions, and includes all algorithms (and/or threshold values) generally known to those skilled in the art.

Once the border is identified, the border-detection algorithm is further adapted to identify at least one control point on the border. For example, with reference to Figures 3 and 4, the border-detection algorithm can be used to identify a plurality of control points 22 on the luminal border 320. It should be appreciated that the location and number of control points depicted in Figure 4 are not intended to limit the present invention, and are merely provided to illustrate the environment in which the present invention may operate. In an alternate example not forming part of the present invention the border-detection application 122 is adapted to identify a border using user-identified control points. Such an embodiment is discussed in detail in U.S. Patent Number 6,381,350, which issued April 30, 2002.

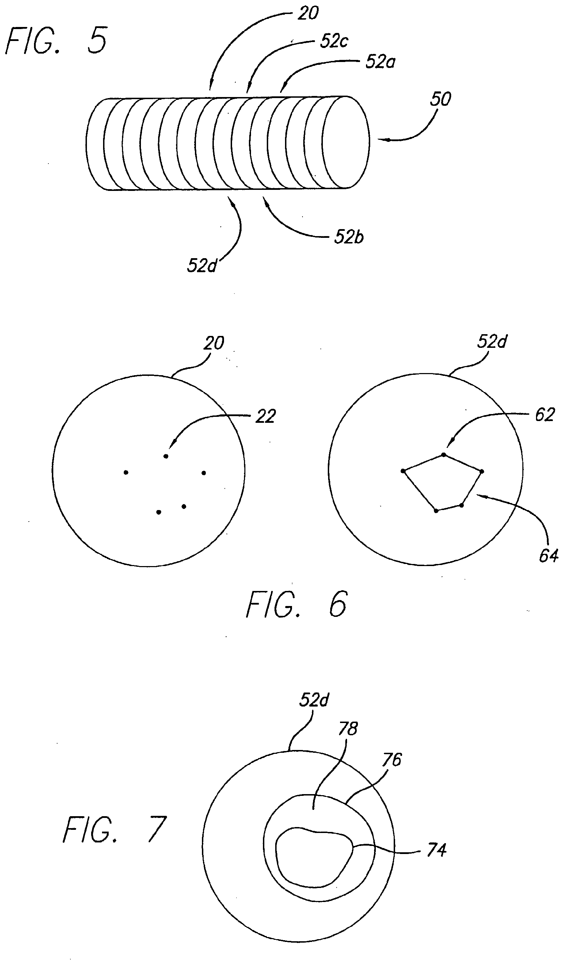

Referring back to Figure 1, once the border and control point(s) are identified on a first vascular image, the extrapolation application 124 is used to identify at least one control point on at least one other IVUS image. In a preferred embodiment of the present invention, this is done by extrapolating the previously identified control points to at least one other IVUS image. By doing this, multiple 2D images (or at least one 3D image) can be produced. For example, as illustrated in Figure 5, multiple 2D images (e.g., 20, 52a-52d, etc.) are used to produce a 3D image of a tubular (e.g., vascular) object 50.

Figure 6 illustrates how an identified control point can be extrapolated to another IVUS image. Specifically, the control points that were illustrated in Figure 4 (i.e., 22) are extrapolated (or copied) to another IVUS image (e.g., 52d), thus creating a second set of control points 62. In one embodiment of the present invention, the control points are extrapolated using Cartesian coordinates. It should be appreciated that, while Figure 6 illustrates control points being extrapolated to an adjacent image, the present invention is not so limited. Thus, extracting control points to additional images (e.g., 52c, 52b, etc.) is within the scope of the present invention.

Once the control points are extrapolated, the extrapolating application is further adapted to identify (or approximate) a border based on the extrapolated points. For example, as shown in Figure 6, the extrapolated points 62 may be connected using a plurality of lines 64, where the lines are either straight or curved (not shown). In another embodiment of the present invention, the extrapolating application is adapted to use an algorithm (e.g., a cubic-interpolation algorithm, etc.) to identify line shape.

Referring back to Figure 1, the active-contour application 126 is then used to adjust the border to more closely match the actual border of the vascular object. In doing so, the active-contour application 126 may consider or take into account at least (i) image gradients (i.e., gradient data), (ii) the proximity of the border to each extrapolated point (i.e., continuity or control-point factor), and/or (iii) border curvature or smoothness (i.e., curvature or boundary factor). Specifically, by considering gradient data (or a gradient factor), the border can be adjusted if the neighboring pixels (as opposed to the pixels of the border) include border characteristics (e.g., a dark-to-light transition, etc.). By considering a continuity or control-point factor, the border can be adjusted so that it passes through each extrapolated point. Furthermore, by considering a curvature or boundary factor, the border can be adjusted to prevent sharp transitions (e.g., corners, etc.). In one embodiment of the present invention, the continuity and curvature factors are also used to connect related borders on adjacent images. It should be appreciated that if multiple factors are being considered, then individual factors may be weighted more heavily than others. This becomes important if the factors produce different results (e.g., the gradient factor suggests adjusting the border away from an extrapolated point, etc.). It should further be appreciated that the active-contour application may also be used to adjust the border identified by the border-detection application. It should also be appreciated that the present invention is not limited to the use of the aforementioned factors for border optimization, and that the use of additional factors (e.g., frequency factor, etc.) to adjust (or optimize) a border is within the scope of the present invention.

In one embodiment of the present invention, the adjusted borders are configured to be manually manipulated. In other words, at least one point on the border can be selected and manually moved to a new location. The active-contour application is then used (as previously discussed) to reconstruct the border accordingly. In another embodiment of the present invention, the active-contour application is further adapted to adjust related borders in adjacent images. This is done by fitting a geometrical model (e.g., a tensor product B-spline, etc.) over the surface of a plurality of related borders (e.g., as identified on multiple IVUS images). A plurality of points on the geometrical model are then parameterized and formulated into a constrained least-squares system of equations. If a point on the border is manually moved, the active-contour application can utilize these equations to calculate a resulting surface (or mesh of control points). The affected borders (e.g., adjacent borders) can then be adjusted accordingly.

Once the border has been sufficiently adjusted, the aforementioned process can be repeated to identify additional borders. In an alternate embodiment of the present invention, multiple borders (e.g., luminal and medial-adventitial borders) are identified concurrently. The multiple border can then be imaged (in either 2D or 3D) and analyzed by either a skilled practitioner or a computer algorithm. For example, as illustrated in Figure 7, the luminal border 74 and the medial-adventitial border 76 can be used (by either a clinician or an algorithm) to identify the plaque-media complex 78 of a vascular object.

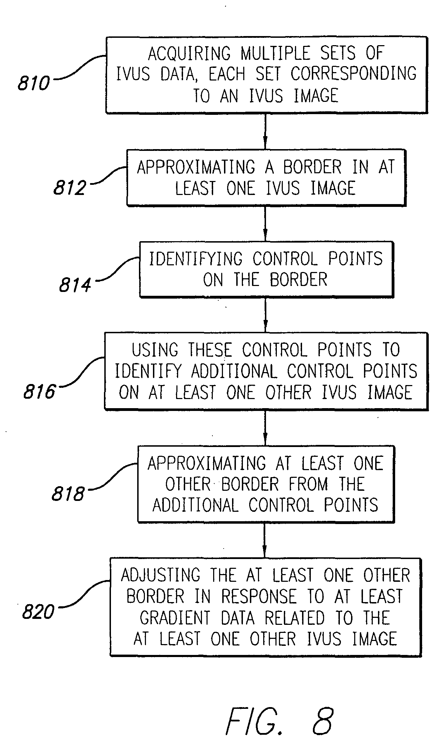

One method of identify a border on a vascular image is illustrated in Figure 8. Specifically, in step 810, multiple sets of IVUS data are acquired, where each set of IVUS data corresponds to a 2D IVUS image. At step 812, a border is approximated in one IVUS image (e.g., using gradient data, etc.). Control points on the approximated border are then identified at step 814. At step 816, these control points are then used to identify additional control points on additional 2D IVUS images (e.g., via extrapolation, etc.). These additional control points are then used to approximate at least one other border at step 818, which is then adjusted at step 820. In one embodiment, the border is adjusted in accordance with at least gradient data.

Having thus described a preferred embodiment of a system and method of identifying a border on a vascular image, it should be apparent to those skilled in the art that certain advantages of the system have been achieved. It should also be appreciated that various modifications, adaptations, and alternative embodiments thereof may be made within the scope of the present invention. The invention is further defined by the following claims.

## Claims

1. A method of identifying a border of a vascular object, comprising:
providing a computing device (120) with multiple sets of blood-vessel data, each set corresponding to an image (20, 52a, 52b, 52c, 52d) of the vascular object;
using a set of blood-vessel data to approximate a border on an image (20) of said vascular object;
identifying, by the computing device, at least one control point (22) on said border;
extrapolating additional control-point data from said at least one control point (22) said additional-control-point data representing at least one other control point (62) on at least one other image (52a, 52b, 52c, 52d);
wherein additional-border data representing at least one other border on said at least one other image is produced from said additional-control-point data; and
adjusting said at least one other border using an active contour application..

2. The method of claim 1, wherein the active contour application adjusts said at least one other border in accordance with at least one factor, said at least one factor being selected from a group consisting of gradient factor, control-point factor and boundary factor, where said control-point factor corresponds to the connectivity of adjacent ones of said additional control points and said boundary factor corresponds to the curvature of said other border.

3. The method of Claim 1, wherein said step of providing a computing device with multiple sets of blood-vessel data further comprises providing a computing device with multiple sets of intra-vascular ultrasound (IVUS) data, where each set corresponds to an IVUS image (20, 52a, 52b, 52c, 52d) of said vascular object.

4. The method of Claim 1, wherein said step of using a set of blood-vessel data to approximate a border further comprises identifying gradients in said image and using said gradients to approximate said border on said image of said vascular object.

5. The method of Claim 1, wherein said step of extrapolating said at least one control point (22) further comprises extrapolating said at least one control point to an adjacent set of blood-vessel data, said adjacent set corresponding to another image adjacent to said image.

6. The method of Claim 1 or 2, wherein said step adjusting said at least one other border further comprises adjusting said at least one other border in accordance with at least a continuity factor, said continuity factor representing an amount of continuity between adjacent control points on said at least one other border.

7. A border-identification system (10) comprising:
a computing device (120) adapted to be electrically connected to a data-gathering device (110) and to acquire from said data-gathering device multiple sets of blood-vessel data, each set corresponding to an image (20, 52a, 52b, 52c, 52d) of a vascular object;
**characterized in**
a border-detection application (122) operating on said computing device (120) and adapted to use at least a portion of said blood-vessel data to produce starting- border data and to identify starting-control-point data, said starting-border data representing at least one border on at least one image (20) of said vascular object and said starting-control-point data representing at least one control point (22) on said at least one border;
an extrapolation application (124) operating on said computing device and adapted to extrapolate additional-control- point data from said starting control-point-data, said additional-control-point data representing at least one other control point (62) on at least one other image (52a, 52b, 52c, 52d), wherein additional-border data representing at least one other border on said at least one other image is produced from said additional-control-point data; and
an active-contour application (126) operating on said computing device and adapted to adjust said at least one other border.

8. The border-identification system of Claim 7, wherein said data-gathering device (110) comprises an intra-vascular ultrasound (IVUS) console.

9. The border-identification system of Claim 7, wherein said border-detection application (122) is further adapted to identify gradients in said at least one image and use said gradients to produce starting-border data.

10. The border-identification system of Claim 9, wherein said border-detection application (122) is further adapted to use said starting-border data to produce said starting-control-point data.

11. The border-identification system of Claim 7, wherein said extrapolation application (124) is further adapted to produce said additional-border data by using cubic interpolation to connect adjacent ones of said at least one other control point.

12. The border-identification system of Claim 7, wherein said active-contour application (126) is further adapted to use gradient data to adjust said at least one other border, said gradient data representing gradients in said at least one other image.

13. The border-identification system of Claim 12, wherein said active-contour application (126) is further adapted to consider the continuity of adjacent ones of said at least one other control point in adjusting said at least one other border.

14. The border-identification system of Claim 13, wherein said active-contour application (126) is further adapted to consider the curvature of said at least one other border in adjusting said at least one other border.

15. The border-identification system of Claim 7, wherein said active-contour application (126) is further adapted to adjust said at least one other border in accordance with at least one factor, said at least one factor being selected from the group consisting of a gradient factor, a continuity factor and a curvature factor.

## Patentansprüche

1. Verfahren zum Identifizieren einer Grenze eines vaskulären Objekts, umfassend:
Bereitstellen einer Rechnervorrichtung (120) mit mehreren Sätzen von Blutgefäßdaten, wobei jeder Satz einem Bild (20, 52a, 52b, 52c, 52d) des vaskulären Objekts entspricht;
Verwenden eines Satzes von Blutgefäßdaten, um eine Grenze auf einem Bild (20) des genannten vaskulären Objekts zu approximieren;
Identifizieren, durch die Rechnervorrichtung, von mindestens einem Kontrollpunkt (22) auf der genannten Grenze;
Extrapolieren von zusätzlichen Kontrollpunktdaten anhand des genannten mindestens einen Kontrollpunkts (22), wobei die genannten zusätzlichen Kontrollpunktdaten mindestens einen weiteren Kontrollpunkt (62) auf mindestens einem weiteren Bild (52a, 52b, 52c, 52d) darstellen;
wobei zusätzliche Grenzdaten, die mindestens eine weitere Grenze auf dem genannten mindestens einen weiteren Bild darstellen, anhand der genannten zusätzlichen Kontrollpunktdaten erzeugt werden; und
Anpassen der genannten mindestens einen weiteren Grenze unter Verwendung einer Aktive-Kontur-Anwendung.

2. Verfahren nach Anspruch 1, wobei die Aktive-Kontur-Anwendung die genannte mindestens eine weitere Grenze in Übereinstimmun mit mindestens einem Faktor anpasst, wobei der genannte mindestens eine Faktor ausgewählt wird aus einer Gruppe bestehend aus Gradientenfaktor, Kontrollpunktfaktor und Begrenzungsfaktor, wobei der genannte Kontrollpunktfaktor der Konnektivität von benachbarten der genannten Kontrollpunkte entspricht und der genannte Begrenzungsfaktor der Krümmung der genannten weiteren Grenze entspricht.

3. Verfahren nach Anspruch 1, wobei der genannte Schritt des Bereitstellens einer Rechnervorrichtung mit mehreren Sätzen von Blutgefäßdaten weiterhin das Bereitstellen einer Rechnervorrichtung mit mehreren Sätzen von intravaskulären Ultraschalldaten (IVUS-Daten) umfasst, wobei jeder Satz einem IVUS-Bild (20, 52a, 52b, 52c, 52d) des genannten vaskulären Objekts entspricht.

4. Verfahren nach Anspruch 1, wobei der genannte Schritt des Verwendens eines Satzes von Blutgefäßdaten zum Approximieren einer Grenze weiterhin das Identifizieren von Gradienten in dem genannten Bild und das Verwenden der genannten Gradienten zum Approximieren der genannten Grenze auf dem genannten Bild des genannten vaskulären Objekts umfasst.

5. Verfahren nach Anspruch 1, wobei der genannte Schritt des Extrapolierens des genannten mindestens einen Kontrollpunkts (22) weiterhin das Extrapolieren des genannten mindestens einen Kontrollpunkts zu einem benachbarten Satz von Blutgefäßdaten umfasst, wobei der benachbarte Satz einem weiteren Bild entspricht, das an das genannte Bild angrenzt.

6. Verfahren nach Anspruch 1 oder 2, wobei der genannte Schritt des Anpassens von mindestens einer weiteren Grenze das Anpassen der genannten mindestens einen weiteren Grenze in Übereinstimmung mit mindestens einem Kontinuitätsfaktor umfasst, wobei der genannte Kontinuitätsfaktor ein Kontinuitätsmaß zwischen benachbarten Kontrollpunkten auf der genannten mindestens einen weiteren Grenze darstellt.

7. Grenzenidentifizierungssystem (10), umfassend:
eine Rechnervorrichtung (120), die dafür ausgelegt ist, mit einer Datenerfassungsvorrichtung (110) elektrisch verbunden zu werden und von der genannten Datenerfassungsvorrichtung mehrere Sätze von Blutgefäßdaten zu erlangen, wobei jeder Satz einem Bild (20, 52a, 52b, 52c, 52d) eines vaskulären Objekts entspricht;
**gekennzeichnet durch**
eine Grenzendetektionsanwendung (122), die auf der genannten Rechnervorrichtung (120) läuft und dafür ausgelegt ist, mindestens einen Teil der genannten Blutgefäßdaten zu verwenden, um Start-Grenzdaten zu erzeugen und um Start-Kontrollpunktdaten zu identifizieren, wobei die genannten Start-Grenzdaten mindestens eine Grenze auf mindestens einem Bild (20) des genannten vaskulären Objekts darstellen und die genannten Start-Kontrollpunktdaten mindestens einen Kontrollpunkt (22) auf der genannten mindestens einen Grenze darstellen;
eine Extrapolationsanwendung (124), die auf der genannten Rechnervorrichtung läuft und dafür ausgelegt ist, zusätzliche Kontrollpunktdaten ausgehend von den genannten Start-Kontrollpunktdaten zu extrapolieren, wobei die genannten zusätzlichen Kontrollpunktdaten mindestens einen weiteren Kontrollpunkt (62) auf mindestens einem weiteren Bild (52a, 52b, 52c, 52d) darstellen, wobei zusätzliche Grenzdaten, die mindestens eine weitere Grenze auf dem genannten mindestens einen weiteren Bild darstellen, anhand der genannten zusätzlichen Kontrollpunktdaten erzeugt werden; und
eine Aktive- Kontur-Anwendung (126), die auf der genannten Rechnervorrichtung läuft und dafür ausgelegt ist, die genannte mindestens eine weitere Grenze anzupassen.

8. Grenzenidentifizierungssystem nach Anspruch 7, wobei die genannte Datenerfassungsvorrichtung (110) eine mit intravaskuläre Ultraschallkonsole (IVUS-Konsole) umfasst.

9. Grenzenidentifizierungssystem nach Anspruch 7, wobei die genannte Grenzendetektionsanwendung (122) weiterhin dafür ausgelegt ist, Gradienten in dem genannten mindestens einen Bild zu identifizieren und die genannten Gradienten zu verwenden, um Start-Grenzdaten zu erzeugen.

10. Grenzenidentifizierungssystem nach Anspruch 9, wobei die genannte Grenzendetektionsanwendung (122) weiterhin dafür ausgelegt ist, die genannten Start-Grenzdaten zu verwenden, um die genannten Start-Kontrollpunktdaten zu erzeugen.

11. Grenzenidentifizierungssystem nach Anspruch 7, wobei die genannte Extrapolationsanwendung (124) weiterhin dafür ausgelegt ist, die genannten zusätzlichen Grenzdaten mithilfe von kubischer Interpolation zu erzeugen, um benachbarte der genannten mindestens einen weiteren Kontrollpunkte zu verbinden.

12. Grenzenidentifizierungssystem nach Anspruch 7, wobei die genannte Aktive-Kontur-Anwendung (126) weiterhin dafür ausgelegt ist, Gradientendaten zum Anpassen der genannten mindestens einen weiteren Grenze zu verwenden, wobei die genannten Gradientendaten Gradienten in dem genannten mindestens einen weiteren Bild darstellen.

13. Grenzenidentifizierungssystem nach Anspruch 12, wobei die genannte Aktive-Kontur-Anwendung (126) weiterhin dafür ausgelegt ist, die Kontinuität von benachbarten der genannten mindestens einen weiteren Kontrollpunkte bei der Anpassung der genannten mindestens einen weiteren Grenze zu berücksichtigen.

14. Grenzenidentifizierungssystem nach Anspruch 13, wobei die genannte Aktive-Kontur-Anwendung (126) weiterhin dafür ausgelegt ist, die Krümmung der genannten mindestens einen weiteren Grenze bei der Anpassung der genannten mindestens einen weiteren Grenze zu berücksichtigen.

15. Grenzenidentifizierungssystem nach Anspruch 7, wobei die genannte Aktive-Kontur-Anwendung (126) weiterhin dafür ausgelegt ist, die genannte mindestens eine weitere Grenze in Übereinstimmung mit mindestens einem Faktur anzupassen, wobei der genannte mindestens eine Faktor ausgewählt wird aus der Gruppe bestehend aus einem Gradientenfaktor, einem Kontinuitätsfaktor und einem Krümmungsfaktor.

## Revendications

1. Procédé d'identification d'un contour d'un objet vasculaire, comprenant :
la fourniture d'un dispositif informatique (120) comportant de multiples ensembles de données sur un vaisseau sanguin, chaque ensemble correspondant à une image (20, 52a, 52b, 52c, 52d) de l'objet vasculaire ;
l'utilisation d'un ensemble de données sur un vaisseau sanguin pour évaluer approximativement un contour d'une image (20) dudit objet vasculaire ;
l'identification, par le dispositif informatique, d'au moins un point de contrôle (22) sur ledit contour ;
l'extrapolation de données de point de contrôle supplémentaires à partir dudit au moins un point de contrôle (22), lesdites données de point de contrôle supplémentaires représentant au moins un autre point de contrôle (62) sur au moins une autre image (52a, 52b, 52c, 52d) ;
dans lequel des données de contour supplémentaires représentant au moins un autre contour sur ladite au moins une autre image sont produites à partir desdites données de point de contrôle supplémentaires ; et
l'ajustement dudit au moins un autre contour en utilisant une application de contour active.

2. Procédé selon la revendication 1, dans lequel l'application de contour active ajuste ledit au moins un autre contour conformément à au moins un facteur, ledit au moins un facteur étant choisi dans un groupe consistant en un facteur de gradient, un facteur de point de contrôle et un facteur de délimitation, dans lequel ledit facteur de point de contrôle correspond à la connectivité des points de contrôle adjacents parmi lesdits points de contrôle supplémentaires et ledit facteur de délimitation correspond à la courbure dudit autre contour.

3. Procédé selon la revendication 1, dans lequel ladite étape consistant à fournir un dispositif informatique comportant de multiples ensembles de données de vaisseau sanguin comprend en outre la fourniture d'un dispositif informatique comportant de multiples ensembles de données d'ultrasons intravasculaires (IVUS), où chaque ensemble correspond à une image IVUS (20, 52a, 52b, 52c, 52d) dudit objet vasculaire.

4. Procédé selon la revendication 1, dans lequel ladite étape consistant à utiliser un ensemble de données de vaisseau sanguin pour évaluer approximativement un contour comprend en outre l'identification de gradients dans ladite image et l'utilisation desdits gradients pour évaluer de façon approximative ledit contour sur ladite image dudit objet vasculaire.

5. Procédé selon la revendication 1, dans lequel ladite étape consistant à extrapoler ledit au moins un point de contrôle (22) comprend en outre l'extrapolation dudit au moins un point de contrôle à un ensemble adjacent de données de vaisseau sanguin, ledit ensemble adjacent correspondant à une autre image adjacente à ladite image.

6. Procédé selon la revendication 1 ou 2, dans lequel ladite étape consistant à ajuster ledit au moins un autre contour comprend en outre l'ajustement dudit au moins un autre contour conformément à au moins un facteur de continuité, ledit facteur de continuité représentant un niveau de continuité entre des points de contrôle adjacents sur ledit au moins un autre contour.

7. Système d'identification de contour (10) comprenant :
un dispositif informatique (120) adapté pour être raccordé électriquement à un dispositif de collecte de données (110) et pour acquérir à partir dudit dispositif de collecte de données, de multiples ensembles de données de vaisseau sanguin, chaque ensemble correspondant à une image (20, 52a, 52b, 52c, 52d) d'un objet vasculaire ;
**caractérisé par**
une application de détection de contour (122) fonctionnant sur ledit dispositif informatique (120) et étant adaptée pour utiliser au moins une partie desdites données de vaisseaux sanguin pour produire des données de contour de départ et pour identifier des données de point de contrôle de départ, lesdites données de contour de départ représentant au moins un contour sur au moins une image (20) dudit objet vasculaire et lesdites données de point de contrôle de départ représentant au moins un point de contrôle (22) sur ledit au moins un contour ;
une application d'extrapolation (124) fonctionnant sur ledit dispositif informatique et étant adaptée pour extrapoler des données de point de contrôle supplémentaires à partir desdites données de point de contrôle de départ, lesdites données de point de contrôle supplémentaires représentant au moins un autre point de contrôle (62) sur au moins une autre image (52a, 52b, 52c, 52d), dans lequel lesdites données de contour supplémentaires représentant au moins un autre contour sur ladite au moins une autre image sont produites à partir desdites données de point de contrôle supplémentaires ; et
une application de contour active (126) fonctionnant sur ledit dispositif informatique et étant adaptée pour ajuster ledit au moins un autre contour.

8. Système d'identification de contour selon la revendication 7, dans lequel ledit dispositif de collecte de données (110) comprend une console d'ultrason intravasculaire (IVUS).

9. Système d'identification de contour selon la revendication 7, dans lequel ladite application de détection de contour (122) est en outre adaptée pour identifier des gradients dans ladite au moins une image et pour utiliser lesdits gradients pour produire des données de contour de départ.

10. Système d'identification de contour selon la revendication 9, dans lequel ladite application de détection de contour (122) est en outre adaptée pour utiliser lesdites données de contour de départ pour produire lesdites données de point de contrôle de départ.

11. Système d'identification de contour selon la revendication 7, dans lequel ladite application d'extrapolation (124) est en outre adaptée pour produire lesdites données de contour supplémentaires en utilisant une interpolation cubique pour raccorder des points de contrôle adjacents dudit au moins un autre point de contrôle.

12. Système d'identification de contour selon la revendication 7, dans lequel ladite application de contour active (126) est en outre adaptée pour utiliser des données de gradient pour ajuster ledit au moins un autre contour, lesdites données de gradient représentant des gradients dans ladite au moins une autre image.

13. Système d'identification de contour selon la revendication 12, dans lequel ladite application de contour active (126) est en outre adaptée pour prendre en compte la continuité des points de contrôle adjacents dudit au moins un autre point de contrôle en ajustant ledit au moins un autre contour.

14. Système d'identification de contour selon la revendication 13, dans lequel ladite application de contour active (126) est en outre adaptée pour prendre en compte la courbure dudit au moins un autre contour en ajustant ledit au moins un autre contour.

15. Système d'identification de contour selon la revendication 7, dans lequel ladite application de contour active (126) est en outre adaptée pour ajuster ledit au moins un autre contour conformément à au moins un facteur, ledit au moins un facteur étant choisi dans le groupe consistant en un facteur de gradient, un facteur de continuité et un facteur de courbure.
